# FASCICULE DE BREVET EUROPEEN

(11) **EP 0 374 015 B1**
(45) Date de publication et mention de la délivrance du brevet: **23.03.1994**
(21) Numéro de dépôt: 89403391.9
(22) Date de dépôt: 07.12.1989
(51) Int. Cl.: C07C 45/00, C07F 13/00, C01G 45/06

(54) **Préparation de composés bromés organiques du manganèse et leur application, notamment à la production de cétones**
Verfahren zur Herstellung von bromierten organischen Mangan-Verbindungen und deren Verwendung, insbesondere für die Herstellung von Ketonen
Process for the preparation of brominated organic manganese compounds and their use, specially in the preparation of ketones

(30) Priorité: 16.12.1988 FR 8816598
(43) Date de publication de la demande: 20.06.1990
(73) Titulaire: SOCIETE NATIONALE ELF AQUITAINE, 92400 Courbevoie (FR)
(72) Inventeur: Cahiez, Gérard, F-75003 Paris (FR); Laboue, Blandine Université Pierre et Marie Curie, F-75252 Paris Cédex 05 (FR); Tozzolino, Pierre, F-64160 Morlaas (FR)
(74) Mandataire: Bertrand, Didier

(56) Documents cités:
- EP-A- 0 283 359
- TETRAHEDRON LETTERS, vol. 30, no. 27, 1989, pages 3545-3546, Maxwell Pergamon MacMillan plc, GB; G. CAHIEZ et al.: "Organomanganese (II) reagents XVII. Preparation of organomanganese bromide compounds in ether: An efficient and economic alternative to organomanganese iodide compounds for synthetic applications"
- TETRAHEDRON LETTERS, vol. 27, no. 37, 1986, pages 4441-4444, Pergamon Journals Ltd, GB; G. CAHIEZ et al.: "Organomanganese (II) reagents XII: An efficient one-pot preparation of unsymmetrical secondary or tertiary alcohols"
- "Gmelin Handbuch der Anorganischen Chemie", édition 8, no. 56, partie C5, 1978, pages 264-292, Springer-Verlag, Berlin, DE; "Verbindungen des Mangans mit Brom und Jod"
- TETRAHEDRON LETTERS, vol. 26, no. 37, 1986, pages 4445-4448, Pergamon Journals Ltd, GB; G. CAHIEZ et al.: "Organomanganese (II) reagents XIII: Highly selective addition of organomanganese halides to aldehydes in the presence of ketones"
- SYNTHESIS, janvier 1984, pages 37-38; G. FRIOUR et al.: "Organomanganous reagents; IX. Preparation of various halogenated, alkoxylated, aryloxylated, and arylsulfenylated ketones from correspondingly functionalized carboxylic acid chlorides or anhydrides"
- TETRAHEDRON LETTERS, no. 36, 1976, pages 3155-3156, Pergamon Press, GB; G. CAHIEZ et al.: "Reactivité des derives organo-manganeux. I-Action sur les chlorures d'acides. Synthese de cetones"

## Description

L'invention se rapporte à la préparation de composés organo-manganeux bromés, ainsi qu'à leurs applications à la synthèse de substances organiques, en particulier de cétones.

Les developpements récents dans la chimie des halogénures de manganèse, portant sur la formation d'organo-manganeux en vue de la synthèse organique, ont surtout concerné MnCl₂ et MnI₂. L'iodure de manganèse, grâce à sa bonne solubilité dans le solvant si courant qu'est l'éther de diéthyle, a été beaucoup employé en recherche de laboratoire : les organo-manganeux mixtes iodés sont préparés rapidement et quantitativement par action d'un organolithien ou d'un organomagnésien sur l'iodure de manganèse MnI₂ au sein de l'éther. Toutefois, l'emploi d'organo-manganeux iodés présente des inconvénients : ils sont préparés à partir d'iodure de manganèse qui est l'halogénure le plus coûteux, et cet iodure n'est pas accessible commercialement à un degré de pureté satisfaisant, ce qui entraîne, dans certains cas, les diminutions significatives de rendement. Pour des raisons industrielles, on a travaillé à partir de chlorure de manganèse bien plus économique et plus stable. Il est toutefois nécessaire d'opérer dans un solvant tel que le tétrahydrofuranne : en effet MnCl₂ est insoluble dans l'éther de diéthyle et il est donc très difficile d'accéder, dans l'éther, aux composés organo-manganeux mixtes chlorés de façon satisfaisante. Quant à MnBr₂, son manque de solubilité dans l'éther de diéthyle n'a pas été surmonté jusqu'à présent. Or, comme on le verra plus loin, il est souvent avantageux d'utiliser, en synthèse organique, les organo-manganeux bromés dans l'éther, les rendements et les sélectivités étant bien meilleurs que dans le tétrahydrofuranne. Cette lacune (l'insolubilité de MnBr₂ dans l'éther) est comblée par la présente invention.

D'après cette invention, le bromure de manganèse est complexé dans un éther acyclique avec des halogénures de lithium, ce qui conduit à des complexes solubles dans des éthers, tels que l'éther de diéthyle, pouvant aisément conduire à des solutions ou à des suspensions d'organo-manganeux bromés dans l'éther ; ces derniers permettent d'obtenir des composés organiques très divers avec d'excellents rendements. Par rapport aux composés iodés, les composés bromés obtenus, suivant l'invention, présentent l'avantage d'être à la fois bien plus économiques et plus stables.

Par rapport aux composés organo-manganeux chlorés, ils permettent certaines synthèses avec de bien meilleurs rendements, quel que soit le solvant utilisé, pour préparer ces composés chlorés : dans le tétrahydrofuranne et, bien entendu, dans l'éther de diéthyle, dans lequel MnCl₂ reste insoluble. Par rapport aux composés manganeux bromés dans le THF, ils permettent également d'obtenir de meilleurs rendements ou de meilleures sélectivités : l'acylation de RMnBr (et de RMnCl) dans le THF donne, dans bien des cas, des rendements qui ne dépassent pas 80%. Dans l'éther, les mêmes réactions avec RMnBr conduisent dans ces mêmes cas à des rendements de l'ordre de 98%. Il est également frappant de constater que l'acylation de RMnBr (et de RMnCl) dans le THF par des anhydrides mixtes (R′COOCOR˝) donne en majeure partie des esters au lieu de la cétone attendue, alors que dans l'éther les mêmes réactions avec RMnBr conduisent à la cétone avec de bons rendements. Enfin les composés bromés suivant l'invention ont permis d'envisager et/ou de réaliser la préparation de certaines cétones élaborées telles que des cétones acétyléniques, par exemple Bu-C≡C-CO-CH=CH-Me, qu'on n'a pas réussi à préparer avec RMnI car il se forme des polymères, ou avec RMnCl, et en particulier des cétones acétyléniques portant des groupes fonctionnels; tels sont par exemple les produits (Et₂O)₂CHC≡C-CO(CH₂)₅OCHO et CH₃OCH₂C=C-CO-5CH₂ )₅Cl.

Ainsi grâce aux composés bromés du manganèse, suivant l'invention, on est actuellement en mesure de faire des produits fins, souvent fragiles, en opérant dans des conditions douces, en général entre -10°C et +20°C, et en particulier entre 0° et 20°C.

Les composés employés, suivant l'invention, sont constitués par des complexes d'une molécule de MnBr₂ avec 1 à 4 moles d'un halogénure de lithium; ce sont des corps de formule générale MnBr₂.nLiX, où X est un halogène, n étant 1 à 4.

Les composés particulièrement efficaces, MnBr₄Li₂ et MnBr₃Li, solubles dans l'éther, réagissent très rapidement dans l'éther avec des organo-lithiens ou organo-magnésiens, pour donner les organo-manganeux bromés correspondants.

Conformément à l'invention, la solution ou suspension dans un éther anhydre, aliphatique, acyclique, liquide, d'un bromure organo-manganeux RMnBr, où R est un groupe , alkyle, alcényle, alcynyle, cyclo-alkyle, cyclo-alcényle ou aryle, pouvant porter un ou plusieurs substituants, ce groupe ayant de préférence 1 à 24 atomes de carbone, la solution ou suspension renfermant au moins 1 mole d'un halogénure de Li par atome de Mn present, est caractérisée en ce qu'elle contient au moins une mole d'halogénure de Mg par atome de Mn présent.

Bien que l'emploi d'organo-lithiums ait été signalé dans la littérature, il n'était pas question de l'utiliser conjointement avec des organo-magnésiens (TETRAHEDRON LETTERS, vol.26, 1986, page 4445-4448, et vol. 27, 1986 pages 4441-4444).

Les halogénures de Mg préférés sont MgCl₂, MgBr₂ et MgC1Br.

La préparation des produits, décrits ci-dessus, consiste à mélanger Mn.Br₂ en poudre avec un halogénure de lithium anhydre, en solution dans un éther aliphatique acyclique, et à agiter le tout jusqu'à dissolution totale ou partielle. Cette opération peut avoir lieu à la température ambiante.

Dans une variante de l'invention, le complexe MnBr₂.nLiX peut être formé in situ, au moment d'une réaction d'utilisation de MnBr₂^{.} On introduit alors le sel de lithium dans un milieu où se trouve un organo-métallique devant réagir avec MnBr₂, celui-ci étant ajouté par la suite.

C'est en particulier le cas de la préparation d'un organo-manganeux RMnBr, qui consiste à dissoudre du LiBr dans une solution éthérée d'un organo-magnésien RMgX, puis à ajouter du MnBr₂ en poudre.

Dans le cas, particulièrement intéressant, où l'halogénure de lithium est le bromure de lithium, il est fort recommandable d'utiliser du LiBr préparé directement dans un éther aliphatique acyclique, par exemple préparé par l'action du brome sur du lithium, au sein de ce solvant, ou bien par la réaction d'un organo-lithium avec un bromure d'alkyle ou un bromure d'allyle. Il est pratique de réaliser ces préparations dans l'éther diéthylique. Elles sont alors conduites à une température voisine de 0°C, notamment -5°C à +5°C, et de préférence à 0°C. On a, dans le premier exemple :

(1) 2Li + Br₂ → 2LiBr

Dans le second exemple, la réaction utilisée peut être :

(2) CH₂=CH-CH₂Br + C₂H₅Li → → LiBr + CH₂=CH-CH₂-C₂H₅

Le bromure de Li, ainsi obtenu, est soluble dans l'éther et forme rapidement un complexe du type MnBr₂LiBr lorsqu'on ajoute, sous agitation, du MnBr₂ en poudre.

Une autre mesure préférée, en vue de la préparation facilitée des complexes suivant l'invention, consiste à employer un MnBr₂ en particules pouvant dépasser 10µ, mais présentant une surface spécifique d'au moins 3,3 m²/g. Un tel sel peut être préparé, conformément à l'invention, par l'action du brome sur une poudre de Mn métallique dans un liquide organique, en particulier un ester aliphatique, de préférence en C₃ à C₉. Dans le cas de l'acétate d'éthyle qui, comme la plupart des solvants courants, ne dissout pas le MnBr₂ formé, celui-ci est récupéré par filtration.

Des échantillons de MnBr₂, obtenus suivant ce trait particulier de l'invention, ont présenté des surfaces spécifiques de 3,4 à 3,9 m²/g contre 3,0 dans le cas du bromure de manganèse commercial (ALFA-INORGANIC, CHEMETALS, STREM) Malgré une granulométrie de 5,9 à 11µ donc plus grosse que celle du produit commercial (5,9µ), ils se dissolvent plus rapidement en présence de BrLi que les bromures de manganèse commerciaux.

L'application des complexes suivant l'invention à la synthèse de différents composés organiques s'effectue au sein d'un éther aliphatique acyclique, en particulier le diéthyl éther, l'éthyl-n.-propyl éther, l'éthyl-iso. propyl éther, le méthyl-n.propyl éther, etc., ou leurs mélanges. Il peut être utile d'employer un cosolvant, par exemple un éther acyclique plus lourd tel que le di n-butyléther.

L'application de ces complexes commence par la formation d'un organo-manganeux bromé par l'action d'un organo-métallique dérivé d'un métal plus électropositif que Mn sur la combinaison MnBr₂.n LiX au sein d'un éther acyclique aliphatique. Ainsi, dans le cas du complexe avec BrLi on a :

(3) MnBr₂.2LiBr + RMgBr → → RMnBr, 2LiBr + MgBr₂

Ce sont les organo-magnésiens et lithiens qui conviennent le mieux. La réaction a lieu entre -10° et +20°C, en particulier entre 0° et +10°C.

La présence de sel de Li exerce une action stabilisatrice, inattendue, sur l'organomanganeux produit. On constate en effet que, par exemple, le bromure de butyl-Mn en présence de bromure de Li ne se décompose pas en 2 heures à la température ambiante ; par contre, en l'absence de LiBr, il commence à se décomposer après 30 minutes à la même température. Il s'ensuit que les composés organo-manganeux, bromés, obtenus par la réaction susindiquée, se comportent mieux, au cours des opérations ultérieures, que ceux qui ne proviennent pas du complexe suivant la demande.

Les organo-manganeux bromés issus du complexe MnBr₂.nLiX conviennent bien aux diverses synthèses connues, dans l'art, notamment avec RMnI ; ainsi, par exemple, peuvent-ils faire l'objet de réactions telles que carbonatation, hydroxy-alkylation, acylation etc., avec les avantages exposés plus haut.

Le groupe R, dans RMnBr, peut être tout groupe provenant d'un organo-lithien ou d'un organo-magnésien tel qu'un groupe alkyle, alcényle, alcynyle, cycloalkyle, cycloalcényle, aryle, portant éventuellement un ou plusieurs substituants. La partie hydrocarbonée de R peut comporter tout nombre d'atomes de carbone voulu et, le plus souvent, 1 à 24. Il en est de même des groupes hydrocarbonés dans les composés que l'on peut faire réagir avec ces organo-manganeux, en particulier des aldéhydes, cétones, anhydrides ou halogénures d'acides.

Diverses synthèses mènent à ce résultat surprenant qu'il est possible de fixer, avec de bons rendements, sur R, des groupes plus ou moins compliqués ou fragiles, porteurs de différentes fonctions, n'ayant pas pu être fixés de façon satisfaisante dans la technique antérieure. Suivant l'invention, le groupe R de l'organo-manganeux bromé peut, lui aussi, porter des substituants fonctionnels avec lesquels la synthèse était difficilement concevable jusqu'à présent. Deux exemples de composés comportant à la fois une triple liaison, un carbonyle et une fonction aldéhyde ou un halogène sont mentionnés au début de la présente description.

A titre d'exemples non limitatifs, les essais suivants illustrent l'invention.

### EXEMPLE 1

### Préparation du complexe MnBr₄Li₂ en solution dans l'éther.

11,2 g de MnBr₂ pur, anhydre, soit 52 mmoles, et 8,7 g de LiBr pur, anhydre, soit 100 mmoles, sont agités avec 80 ml d'éther anhydre, à la température ambiante, jusqu'à obtention d'une solution limpide.

### EXEMPLE 2

### Préparation du complexe MnBr₃Li en solution dans l'éther

Le mode opératoire est le même que dans l'exemple 1, sauf que 4,35 g de LiBr sont utilisés, soit 50 mmoles.

### EXEMPLE 3

### Préparation de LiBr

Dans 80 ml d'éther anhydre, on introduit 0,7 g de Li métal, soit 0,1 atome-gramme, et l'on ajoute lentement du brome, en agitant à 0°C. Le débit de Br₂ est réglé de façon à ne pas dépasser cette température. La quantité totale de Br₂ introduit est de 8g, soit 50 mmoles lorsque le Li a totalement disparu.

### EXEMPLE 4

### Préparation de MnBr₂

Le mode opératoire préféré, en vue de l'obtention d'un sel particulièrement réactif, de surface spécifique dépassant 3,3 m²/g est conduit comme suit. 110 g de Mn métal (pureté 99%) en poudre, c'est-à-dire 2 atomes-grammes, sont mis en suspension dans 1500 ml d'acétate d'éthyle par vive agitation. On ajoute alors du brome, goutte-à-goutte, jusqu'à un total de 323,2g, soit 2,02 moles de Br₂. Durant toute l'opération, le milieu réactionnel est refroidi, de façon à ne pas dépasser 35°C. L'agitation est continuée pendant 4 heures à 30°C.

Le MnBr₂ précipité est alors séparé par filtration sur verre fritté n^{o}3 et lavé plusieurs fois avec de l'acétate d'éthyle ; il est ensuite placé dans un dessicateur chauffant où le solvant restant est éliminé par chauffage progressif, pendant 6 heures, jusqu'à 200°C sous une pression réduite à 10⁻² torr. Le produit est alors broyé et à nouveau séché sous vide à 200°C. Rendement 95%.

### EXEMPLE 5

### Préparation d'un organo-manganeux bromé

I.- 50 mmoles d'organomagnésien RMgBr, en solution dans 80 ml d'éther, sont mélangées, à 0°C, avec 50 mmoles du complexe MnBr₄Li₂ en solution dans 80 ml d'éther, préparé selon l'exemple 1. Après 1 heure à +10°C, on obtient une suspension de RMnBr dont la couleur varie du marron foncé au vert, suivant la nature du groupe R.
   L'opération a été effectuée notamment pour des RMnBr dont le groupe R est un CH₃, C₄H₉-, C₄H₉-C≡C-, (CH₃)₂C=CH et (C₂H₅)₂CH-C≡C-, avec des rendements supérieurs à 95%. Dans le cas d'un phényle, comme R, il a fallu maintenir le mélange à +10°C pendant 4 heures.
II.- Les opérations de la partie I de l'exemple étant répétées avec 50 mmoles d' organo-lithien RLi à la place du magnésien, des résultats équivalents sont obtenue.

### EXEMPLE 6

### Variante de prépartion d'un organo-manganeux bromé

A 80 ml de solution de 50 mmoles d'organo-magnésien RMgBr dans l'éther, on ajoute 8,7 g de LiBr pur, anhydre, soit 100 mmoles. Après 45 mn d'agitation à la température ambiante, on obtient une solution limpide. Celle-ci est refroidie à -30°C et additionnée de 11,2 g MnBr₂ en poudre, c'est-à-dire 52 mmoles ; puis on agite entre 0°C et l'ambiante. Pour la plupart des groupes R, mentionnés dans l'exemple 5, l'organo-manganeux est en général obtenu en moins d'une heure avec des rendements de 96 à 99% ; dans le cas du phényle, il faut environ 3 h d'agitation.

### EXEMPLE 7

### Exemple Comparatif à la préparation selon exemples 5 et 6.

Les préparations sont effectuées à partir de composés organo-magnésiens, de façon analogue à celles des exemples 5^{I} et 6, mais au lieu de la solution du complexe MnBr₄Li₂ on emploie une suspension de 50 mmoles de MnBr₂ , sans sel de lithium, dans 80 ml d'éther.

Il faut alors agiter le mélange pendant 8 heures, au lieu d'une heure, et le rendement en RMnBr obtenu est de 30 à 80%, alors qu'il est supérieur à 95% dans les exemples précédents.

De plus ces proportions ne sont pas reproductibles et le rendement obtenu est aléatoire.

### EXEMPLES 8 à 11

### Application à la préparation de cétones

Différents organo-manganeux bromés RMnBr, comme ceux qui ont été préparés d'après les exemples 5 et 6, ont été employés à la synthèse de cétones. Pour cela, à une solution ou à une suspension dans 100 ml d'éther à -20°C de 52mmoles de RMnBr on ajoutait 50 mmoles d'un chlorure d'acide R′COCl, en agitant.

La température du milieu réactionnel était ensuite ramenée à l'ambiante et l'on continuait l'agitation durant 2 heures. Le milieu est alors traité avec 60 ml d'HCl 1N à -10°C, puis ramené à température ambiante, après quoi, la phase aqueuse, décantée, est soumise à l'extraction, avec 2 fois 50 ml d'éther. Les phases éthérées réunies sont lavées avec 50 ml d'une solution aqueuse, saturée, de NaHCO₃. Après séchage sur MgSO₄ et évaporation du solvant sous vide, la cétone formée est isolée par distillation.

Le Tableau, qui suit, donne les rendements en cétone obtenus dans ces préparations pour différents groupes organiques R et R′. Ce sont des moyennes des résultats d'essais effectués avec des RMnBr. préparés par les deux modes opératoires des exemples 5I et II.

Dans la dernière colonne verticale figurent les rendements obtenus à partir du RMnBr préparé selon la technique antérieure (exemple 7), c'est-à-dire sans adjonction de LiBr (les rendements sont de plus aléatoires, les réactions étant non reproductibles.

| Exemple n^{o} | R | R′ | Rendements % | |
|---|---|---|---|---|
| | | | Invention | Art antérieur |
| 8 | Bu- | Bu- | 86 | 54 |
| 9 | Ph- | Bu- | 96 | 65 |
| 10 | Bu- | Hept- | 91 | 76 |
| 11 | BuC≡C- | Bu- | 88 | 41 |

Il est clair que les organo-manganeux bromés, produits suivant l'invention, fournissent de meilleurs rendements que ceux de la technique antérieure. De plus, les résultats ci-dessus ont été obtenus en deux heures d'acylation, alors qu'il faut 24 heures pour arriver à 77% de rendement en la phényl butyl cétone, Ph-CO-Bu, par la réaction de Ph-MnCl + BuCOCl, dans l'éther, dans les conditions décrites plus haut (comparer avec l'exemple 9).

### EXEMPLE 12

### Application à la préparation d'une cétone particulièrement fragile

Par la méthode de l'exemple 5, on a préparé l'organo-manganeux BuC≡C-MnBr que l'on a fait ensuite réagir avec le chlorure d'acide MeCH=CH-COCl selon le mode opératoire des exemples 8 à 11, dans l'éther.

On a ainsi obtenu la vinyl alcynyl cétone

BuC≡C-CO-CH=CH-Me

avec un rendement de 87%, alors qu'en partant de BuC≡C-MnI ce sont des polymères qui se forment seulement. Avec le composé chloré correspondant, le rendement (même dans le THF) est très faible.

### EXEMPLE 13

### Préparation du complexe MnBr₄Li₂ à partir de LiBr commercial.

En opérant comme dans l'exemple 1, mais avec du LiBr commercial (marque "FLUKA"), tel quel, au lieu de LiBr obtenu selon l'exemple 3, on n'a pas observé la dissolution des deux sels dans l'éther, après l'agitation du mélange pendant 3 heures à la température ambiante. Par contre, ce bromure de lithium se dissolvait bien dans une solution étherée d'organo-magnésien RMgBr.

### EXEMPLE 14

### Préparation directe d'un organo-manganeux mixte RMnBr à partir d'un magnésien et de LiBr.

52 mmoles de RMgBr (R comme dans l'exemple 5) sont dissous dans 80 ml d'éther anhydre ; on ajoute 100 mmoles de LiBr de commerce et l'on agite à 20°C pendant 1 heure, jusqu'à dissolution de ce sel. La suite est conduite comme dans l'exemple 6 où a été employé un LiBr préparé au laboratoire, pur, anhydre.
On obtient du RMnBr tout comme dans l'exemple 6.

## Revendications (Revendications pour l'(les) Etat(s) contractant(s) suivant(s): BE, CH, DE, GB, GR, IT, LI, LU, NL, SE)

1. Solution ou suspension dans un éther anhydre, aliphatique, acyclique, liquide, d'un bromure organo-maganeux RMnBr, où R est un groupe alkyle, alcényle, alcynyle, cyclo-alkyle, cycloalcényle ou aryle, pouvant porter un ou plusieurs substitutants, ce groupe ayant de préférence 1 à 24 atomes de carbone, la solution ou suspension, renfermant au moins 1 mole d'un halogénure de Li par atome de Mn présent, caractérisée en ce qu'elle contient au moins une mole d'halogénure de Mg par atome de Mn présent.

2. Solution ou suspension suivant la revendication 1, dans laquelle l'halogénure de Mg est MgCl₂, MgBr₂ ou MgBrCl.

3. Solution ou suspension suivant la revendication 1 ou 2 caractérisée en ce que l'éther comporte 2 à 5 atomes de carbone.

4. Procédé de préparation d'une solution ou suspension éthérée d'un bromure organo-manganeux suivant une des revendications précédentes, caractérisé en ce que l'on dissout d'abord du MnBr₂ dans l'éther anhydre, aliphatique, acyclique, liquide, par son agitation avec au moins 1 mole de LiX où X est Cl, Br ou I et addition subséquente d'un organo-métallique RMgX, ou RLi et RMgX.

5. Procédé de préparation d'une solution ou suspension éthérée d'un bromure organo-maganeux suivant une des revendications 1 à 3 caractérisé en ce que l'on fait agiter d'abord une solution de RMgX dans l'éther avec au moins 1 mole de LiX, jusqu'à dissolution de ce dernier, X étant Cl, Br ou I, après quoi on ajoute à la solution du MnBr₂ en quantité molaire égale ou voisine à celle de RMgX mis en oeuvre.

6. Procédé suivant la revendication 4 ou 5, caractérisé en ce que l'halogénure de lithium, en particulier LiBr, est au préalable préparé dans un éther acyclique aliphatique liquide, en particulier par l'action de l'halogène sur le lithium, dans l'éther anhydre, en dessous de la température ambiante, ou par l'action d'un dérivé organique du lithium sur un halogénure d'alkyle ou un halogénure d'allyle.

7. Procédé suivant une des revendications 4 à 6, caractérisé en ce que le bromure manganeux est préparé par l'action du brome sur une poudre de Mn métallique, au sein d'un liquide organique, de préférence un ester aliphatique, notamment l'acétate d'éthyle, la poudre de MnBr₂, ainsi préparée, ayant une surface spécifique supérieure à 3,3 m2/g.

8. Application d'une solution ou suspension selon une des revendications 1 à 3, à la préparation d'un composé organique, par l'agitation de cette solution ou suspension avec un précurseur dudit composé, capable de réagir avec RMnBr.

9. Application suivant la revendication 8, caractérisée en ce que ladite préparation est une carbonatation, hydroxy-alkylation ou acylation du précurseur.

10. Application suivant la revendication 9, caractérisée en ce que le précurseur est un chlorure d'acide organique R'COCl ou un anhydride mixte R'-COO-COR'', R' et R'' étant des groupes hydrocarbonés et le composé organique produit est une cétone.

11. Application suivant la revendication 10, caractérisée en ce que l'organo-manganeux est :
(C₂H₅O)₂-CH-C≡C.MnBr ou CH₃-0-CH₂C≡C-Mn-Br et que le chlorure d'acide est C1C0(CH₂)₅-O-CHO ou C1CO(CH₂)₅Cl.

12. Nouvelles cétones obtenues suivant la revendication 11.

## Revendications (Revendications pour l'(les) Etat(s) contractant(s) suivant(s): AT)

1. Procédé de préparation d'une solution ou suspension étherée d'un bromure organo-manganeux, caractérisé en ce que l'on dissout du MnBr₂ dans l'éther anhydre, aliphatique, acyclique, liquide, par son agitation avec au moins 1 mole de LiX où X est Cl, Br ou I, et addition d'un organo-métallique RMgX, ou RLi et RMgX.

2. Procédé de préparation d'une solution ou suspension étherée d'un bromure organo-manganeux suivant la revendication 1, caractérisé en ce que l'on fait agiter d'abord une solution de RMgX dans l'éther avec au moins 1 mole de LiX, jusqu'à dissolution de ce dernier, X étant Cl, Br ou I, après quoi on ajoute à la solution du MnBr₂ en quantité molaire égale ou voisine à celle de RMgX mis en oeuvre.

3. Procédé suivant la revendication 1 ou 2, caractérisé en ce que l'halogénure de lithium, en particulier LiBr, est au préalable préparé dans un éther acyclique aliphatique liquide, en particulier par l'action de l'halogène sur le lithium, dans un éther anhydre, en dessous de la température ambiante, ou par l'action d'un dérivé organique du lithium sur un halogénure d'alkyle ou un halogénure d'allyle.

4. Procédé suivant une des revendications 1 à 3, caractérisé en ce que le bromure manganeux est préparé par l'action du brome sur une poudre de Mn métallique, au sein d'un liquide organique, de préférence un ester aliphatique, notamment l'acétate d'éthyle, la poudre de MnBr₂, ainsi préparée, ayant une surface spécifique supérieure à 3,3 m²/g.

5. Application du procédé selon une des revendications 1 à 4, à la préparation d'un composé organique, caractérisée en ce que l'on fait agiter la solution ou suspension obtenue avec un précurseur du dit composé, capable de réagir avec RMnBr.

6. Application suivant la revendication 5, caractérisée en ce que ladite préparation est une carbonatation, hydroxy-alkylation ou acylation du précurseur.

7. Application suivant la revendication 6, caractérisée en ce que précurseur est un chlorure d'acide organique R′COCl, ou un anhydride mixte R′-COO-COR˝, R′ et R˝ étant des groupes hydrocarbonés et le composé organique produit est une cétone.

8. Application suivant la revendication 7, caractérisée en ce que l'organo-manganeux est :
(C₂H₅O)₂-CH-C≡C-MnBr ou CH₃-O-CH₂-C≡C-Mn-Br et que le chlorure d'acide est ClCO(CH₂)₅-O-CHO ou ClCO(CH₂)₅Cl.

## Revendications (Revendications pour l'(les) Etat(s) contractant(s) suivant(s): ES)

1. Procédé de préparation d'une solution ou suspension étherée d'un bromure organo-manganeux, caractérisé en ce que l'on dissout du MnBr₂ dans l'éther anhydre, aliphatique, acyclique, liquide, par son agitation avec au moins 1 mole de LiX où X est Cl, Br ou I, et addition d'un organo-métallique RMgX, ou RLi et RMgX.

2. Procédé de préparation d'une solution ou suspension étherée d'un bromure organo-manganeux suivant la revendication 1, caractérisé en ce que l'on fait agiter d'abord une solution de RMgX dans l'éther avec au moins 1 mole de LiX, jusqu'à dissolution de ce dernier, X étant Cl, Br ou I, après quoi on ajoute à la solution du MnBr₂ en quantité molaire égale ou voisine à celle de RMgX mis en oeuvre.

3. Procédé suivant la revendication 1 ou 2, caractérisé en ce que l'halogénure de lithium, en particulier LiBr, est au préalable préparé dans un éther acyclique aliphatique liquide, en particulier par l'action de l'halogène sur le lithium, dans un éther anhydre, en dessous de la température ambiante, ou par l'action d'un dérivé organique du lithium sur un halogénure d'alkyle ou un halogénure d'allyle.

4. Procédé suivant une des revendications 1 à 3, caractérisé en ce que le bromure manganeux est préparé par l'action du brome sur une poudre de Mn métallique, au sein d'un liquide organique, de préférence un ester aliphatique, notamment l'acétate d'éthyle, la poudre de MnBr₂, ainsi préparée, ayant une surface spécifique supérieure à 3,3 m²/g.

5. Application du procédé selon une des revendications 1 à 4, à la préparation d'un composé organique, caractérisée en ce que l'on fait agiter la solution ou suspension obtenue avec un précurseur du dit composé, capable de réagir avec RMnBr.

6. Application suivant la revendication 5, caractérisée en ce que ladite préparation est une carbonatation, hydroxy-alkylation ou acylation du précurseur.

7. Application suivant la revendication 6, caractérisée en ce que précurseur est un chlorure d'acide organique R′COCl, ou un anhydride mixte R′-COO-COR˝, R′ et R˝ étant des groupes hydrocarbonés et le composé organique produit est une cétone.

8. Application suivant la revendication 7, caractérisée en ce que l'organo-manganeux est :
(C₂H₅O)₂-CH-C≡C-MnBr ou CH₃-O-CH₂-C≡C-Mn-Br et que le chlorure d'acide est ClCO(CH₂)₅-O-CHO ou ClCO(CH₂)₅Cl.

## Claims (Claims for the following Contracting State(s): BE, CH, DE, GB, GR, IT, LI, LU, NL, SE)

1. Solution or suspension in a liquid acyclic aliphatic anhydrous ether of an organo-manganese bromide RMnBr, where R is an alkyl, alkenyl, alkynyl, cycloalkyl, cycloalkenyl or aryl group, capable of supporting one or several substituents, this group preferably having 1 to 24 carbon atoms, the solution or suspension including at least one mole of Li halide per Mn atom present, characterised in that it contains at least one mole of Mg halide per Mn atom present.

2. Solution or suspension according to Claim 1, in which the Mg halide is MgCl₂, MgBr₂ or MgBrCl.

3. Solution or suspension according to Claim 1 or 2, characterised in that the ether has 2 to 5 carbon atoms.

4. Process for preparation of an ethereal solution or suspension of an organo-manganese bromide according to one of the preceding claims, characterised in that first of all MnBr₂ is dissolved in liquid acyclic aliphatic anhydrous ether by agitation with at least 1 mole of LiX where X is Cl, Br or I, and subsequent addition of an organometal RMgX, or RLi and RMgX.

5. Process for preparation of an ethereal solution or suspension of an organo-manganese bromide according to one of Claims 1 to 3, characterised in that first of all a solution of RMgX is agitated in the ether with at least 1 mole of LiX until the latter dissolves, X being Cl, Br or I, after which MnBr₂ is added to the solution in a molar quantity equal or close to that of the RMgX employed.

6. Process according to Claim 4 or 5, characterised in that the lithium halide, particularly LiBr, is previously prepared in a liquid aliphatic acyclic ether, particularly by the action of the halogen on the lithium, in the anhydrous ether, below the ambient temperature, or by the action of an organic derivative of lithium on an alkyl halide or an allyl halide.

7. Process according to one of Claims 4 to 6, characterised in that the manganese bromide is prepared by the action of the bromine on a metal Mn powder, within an organic liquid, preferably an aliphatic ester, notably ethyl acetate, the MnBr₂ powder thus prepared having a specific surface greater than 3.3 m²/g.

8. Application of a solution or suspension according to one of Claims 1 to 3 to the preparation of an organic compound, by the agitation of this solution or suspension with a precursor of the said compound capable of reacting with RMnBr.

9. Application according to Claim 8, characterised in that the said preparation is a carbonation, hydroxy-alkylation or acylation of the precursor.

10. Application according to Claim 9, characterised in that the precursor is an organic acid chloride R'COCl or a mixed anhydride R'-COO-COR", R' and R'' being hydrocarbon groups and the organic compound produced is a ketone.

11. Application according to Claim 10, characterised in that the organo-manganese is: (C₂H₅O)₂-CH-C≡C.MnBr or CH₃-O-CH₂-C≡C-Mn-Br and the acid chloride is ClCO(CH₂)₅-O-CHO or ClCO(CH₂)₅Cl.

12. Novel ketones obtained according to Claim 11.

## Claims (Claims for the following Contracting State(s): AT)

1. Process for preparation of an ethereal solution or suspension of an organo-manganese bromide, characterised in that MnBr₂ is dissolved in liquid acyclic aliphatic anhydrous ether by agitation with at least 1 mole of LiX where X is Cl, Br or I, and addition of an organometal RMgX, or RLi and RMgX.

2. Process for preparation of an ethereal solution or suspension of an organo-manganese bromide according to Claim 1, characterised in that first of all a solution of RMgX is agitated in the ether with at least 1 mole of LiX until the latter dissolves, X being Cl, Br or I, after which MnBr₂ is added to the solution in a molar quantity equal or close to that of the RMgX employed.

3. Process according to Claim 1 or 2, characterised in that the lithium halide, particularly LiBr, is previously prepared in a liquid aliphatic acyclic ether, particularly by the action of the halogen on the lithium, in an anhydrous ether, below the ambient temperature, or by the action of an organic derivative of lithium on an alkyl halide or an allyl halide.

4. Process according to one of Claims 1 to 3, characterised in that the manganese bromide is prepared by the action of the bromine on a metal Mn powder, within an organic liquid, preferably an aliphatic ester, notably ethyl acetate, the MnBr₂ powder thus prepared having a specific surface greater than 3.3 m²/g.

5. Application of the process according to one of Claims 1 to 4 to the preparation of an organic compound, characterised in that the solution or suspension obtained is agitated with a precursor of the said compound capable of reacting with RMnBr.

6. Application according to Claim 5, characterised in that the said preparation is a carbonation, hydroxy-alkylation or acylation of the precursor.

7. Application according to Claim 6, characterised in that the precursor is an organic acid chloride R'COCl or a mixed anhydride R'-COO-COR'', R' and R'' being hydrocarbon groups and the organic compound produced is a ketone.

8. Application according to Claim 7, characterised in that the organo-manganese is:
(C₂H₅O)₂-CH-C≡C-MnBr or CH₃-O-CH₂-C≡C-Mn-Br and the acid chloride is ClCO(CH₂)₅-O-CHO or ClCO(CH₂)₅Cl.

## Claims (Claims for the following Contracting State(s): ES)

1. Process for preparation of an ethereal solution or suspension of an organo-manganese bromide, characterised in that MnBr₂ is dissolved in liquid acyclic aliphatic anhydrous ether by agitation with at least 1 mole of LiX where X is Cl, Br or I, and addition of an organometal RMgX, or RLi and RMgX.

2. Process for preparation of an ethereal solution or suspension of an organo-manganese bromide according to Claim 1, characterised in that first of all a solution of RMgX is agitated in the ether with at least 1 mole of LiX until the latter dissolves, X being Cl, Br or I, after which MnBr₂ is added to the solution in a molar quantity equal or close to that of the RMgX employed.

3. Process according to Claim 1 or 2, characterised in that the lithium halide, particularly LiBr, is previously prepared in a liquid aliphatic acyclic ether, particularly by the action of the halogen on the lithium, in an anhydrous ether, below the ambient temperature, or by the action of an organic derivative of lithium on an alkyl halide or an allyl halide.

4. Process according to one of Claims 1 to 3, characterised in that the manganese bromide is prepared by the action of the bromine on a metal Mn powder, within an organic liquid, preferably an aliphatic ester, notably ethyl acetate, the MnBr₂ powder thus prepared having a specific surface greater than 3.3 m²/g.

5. Application of the process according to one of Claims 1 to 4 to the preparation of an organic compound, characterised in that the solution or suspension obtained is agitated with a precursor of the said compound capable of reacting with RMnBr.

6. Application according to Claim 5, characterised in that the said preparation is a carbonation, hydroxy-alkylation or acylation of the precursor.

7. Application according to Claim 6, characterised in that the precursor is an organic acid chloride R'COCl or a mixed anhydride R'-COO-COR'', R' and R'' being hydrocarbon groups and the organic coipound produced is a ketone.

8. Application according to Claim 7, characterised in that the organo-manganese is:
(C₂H₅O)₂-CH-C≡C-MnBr or CH₃-O-CH₂-C≡C-Mn-Br and the acid chloride is ClCO(CH₂)₅-O-CHO or ClCO(CH₂)₅Cl.

## Patentansprüche (Patentansprüche für folgende(n) Vertragsstaat(en): BE, CH, DE, GB, GR, IT, LI, LU, NL, SE)

1. Lösung oder Suspension in einem wasserfreien aliphatischen acyclischen flüssigen Ether eines Organo-Manganbromids RMnBr, worin R für eine Alkyl-, Alkenyl-, Alkynyl-, Cycloalkyl-, Cylcoalkenyl- oder Aryl-Gruppe steht, die einen oder mehrere Substituenten tragen kann, wobei diese Gruppe vorzugsweise 1 bis 24 Kohlenstoffatome aufweist, die Lösung oder Suspension mindestens 1 Mol Li-Halogenid pro vorhandenem Mn-Atom enthält, dadurch gekennzeichnet, daß sie mindestens 1 Mol Mg-Halogenid pro vorhandenem Mn-Atom enthält.

2. Lösung oder Suspension nach Anspruch 1, worin das Mg-Halogenid MgCl₂, MgBr₂ oder MgBrCl ist.

3. Lösung oder Suspension nach Anspruch 1 oder 2, dadurch gekennzeichnet, daß der Ether 2 bis 5 Kohlenstoffatome aufweist.

4. Verfahren zur Herstellung einer etherischen Lösung oder Suspension eines Organo-Manganbromids nach einem der vorgenannten Ansprüche, dadurch gekennzeichnet, daß zuerst MnBr₂ in wasserfreiem aliphatischem acyclischem flüssigem Ether aufgelöst wird, indem er mit mindestens 1 Mol LiX, worin X für Cl, Br oder I steht, geschüttelt wird und anschließend organo-metallisches RMgX oder RLi und RMgX hinzugegeben werden.

5. Verfahren zur Herstellung einer etherischen Lösung oder Suspension eines Organo-Manganbromids nach Anspruch 1, dadurch gekennzeichnet, daß zunächst eine Lösung aus RMgX in Ether, wobei X für Cl, Br oder I steht, mit mindestens 1 Mol LiX gerührt wird, bis sich letzteres aufgelöst hat, worauf zu der Lösung MnBr₂ in einer molaren Menge hinzugegeben wird, die gleich ist wie die des hergestellten RMgX oder in deren Nähe liegt.

6. Verfahren nach Anspruch 4 oder 5, dadurch gekennzeichnet, daß das Lithiumhalogenid, insbesondere LiBr, zuvor hergestellt wird in einem acyclischen aliphatischen flüssigen Ether, insbesondere unter Einwirken von Halogen auf Lithium in einem wasserfreien Ether unterhalb von Umgebungstemperatur, oder durch Einwirken eines organischen Lithiumderivats auf ein Alkyl- oder Allylhalogenid.

7. Verfahren nach einem der Ansprüche 4 bis 6, dadurch gekennzeichnet, daß das Manganbromid hergestellt wird durch Einwirken von Brom auf metallisches Mn-Pulver in einem organischen Lösungsmittel, vorzugsweise in einem aliphatischen Ester, insbesondere Ethylacetat, wobei das so hergestellte MnBr₂-Pulver eine spezifische Oberfläche von über 3,3 m²/g aufweist.

8. Verwendung einer Lösung oder Suspension nach einem der Ansprüche 1 bis 3 zur Herstellung einer organischen Verbindung, indem diese Lösung oder Suspension, erhalten mit einer Vorstufe der genanntenVerbindung, die in der Lage ist, mit RMnBr zu reagieren, gerührt wird.

9. Anwendung nach Anspruch 8, dadurch gekennzeichnet, daß die genannte Herstellung eine Carbonatisierung, Hydroxyalkylierung oder Acylierung der Vorstufe ist.

10. Anwendung nach Anspruch 9, dadurch gekennzeichnet, daß die Vorstufe ein organisches Säurechlorid R'COCl oder ein gemischtes Anhydrid R'-COO-COR'' ist, wobei R' und R'' Kohlenwasserstoff-Gruppen darstellen und die hergestellte organische Verbindung ein Keton ist.

11. Anwendung nach Anspruch 10, dadurch gekennzeichnet, daß das Organo-Mangan folgendes ist:
(C₂H₅O)₂-CH-C≡C-Mn-Br oder CH₃-O-CH₂C≡C-Mn-Br und daß das Säurechlorid ClCO(CH₂)₅-O-CHO oder ClCO(CH₂)₅Cl ist.

12. Neue Ketone, erhalten nach Anspruch 11.

## Patentansprüche (Patentansprüche für folgende(n) Vertragsstaat(en): AT)

1. Verfahren zur Herstellung einer etherischen Lösung oder Suspension eines Organo-Manganbromids, dadurch gekennzeichnet, daß MnBr₂ in wasserfreiem aliphatischem acyclischem flüssigem Ether aufgelöst wird, indem er mit mindestens 1 Mol LiX, worin X für Cl, Br oder I steht, geschüttelt wird und organo-metallisches RMgX oder RLi und RMgX hinzugegeben werden.

2. Verfahren zur Herstellung einer etherischen Lösung oder Suspension eines Organo-Manganbromids nach Anspruch 1, dadurch gekennzeichnet, daß zunächst eine Lösung aus RMgX in Ether, wobei X für Cl, Br oder I steht, mit mindestens 1 Mol LiX gerührt wird, bis sich letzteres aufgelöst hat, worauf zu der Lösung MnBr₂ in einer molaren Menge hinzugegeben wird, die gleich ist wie die des hergestellten RMgX oder in deren Nähe liegt.

3. Verfahren nach Anspruch 1 oder 2, dadurch gekennzeichnet, daß das Lithiumhalogenid, insbesondere LiBr zuvor hergestellt wird in einem acyclischen aliphatischen flüssigen Ether, insbesondere unter Einwirken des Halogens auf Lithium in einem wasserfreien Ether unterhalb von Umgebungstemperatur, oder durch Einwirken eines organischen Lithiumderivats auf ein Alkyl- oder Allylhalogenid.

4. Verfahren nach einem der Ansprüche 1 bis 3, dadurch gekennzeichnet, daß das Manganbromid hergestellt wird durch Einwirken von Brom auf metallisches Mn-Pulver in einer organischen Flüssigkeit, vorzugsweise in einem aliphatischen Ester, insbesondere Ethylacetat, wobei das so hergestellte MnBr₂-Pulver eine spezifische Oberfläche von über 3,3 m²/g aufweist.

5. Anwendung des Verfahrens nach einem der Ansprüche 1 bis 4 zur Herstellung einer organischen Verbindung, dadurch gekennzeichnet, daß die Lösung oder Suspension, erhalten mit einer Vorstufe der genannten Verbindung, die in der Lage ist, mit RMnBr zu reagieren, gerührt wird.

6. Anwendung nach Anspruch 5, dadurch gekennzeichnet, daß die genannte Herstellung eine Carbonatisierung, Hydroxyalkylierung oder Acylierung der Vorstufe ist.

7. Anwendung nach Anspruch 6, dadurch gekennzeichnet, daß die Vorstufe ein organisches Säurechlorid R'-COCl oder einer gemischtes Anhydrid R'-COO-COR'' ist, wobei R' und R'' Kohlenwasserstoff-Gruppen darstellen und die hergestellte organische Verbindung ein Keton ist.

8. Anwendung nach Anspruch 7, dadurch gekennzeichnet, daß das Organo-Mangan folgendes ist:
(C₂H₅O)₂-CH-C≡C-Mn-Br oder CH₃-O-CH₂C≡C-Mn-Br und daß das Säurechlorid ClCO(CH₂)₅-O-CHO oder ClCO(CH₂)₅Cl ist.

## Patentansprüche (Patentansprüche für folgende(n) Vertragsstaat(en): ES)

1. Verfahren zur Herstellung einer etherischen Lösung oder Suspension eines Organo-Manganbromids, dadurch gekennzeichnet, daß MnBr₂ in wasserfreiem aliphatischem acyclischem flüssigem Ether aufgelöst wird, indem er mit mindestens 1 Mol LiX, worin X für Cl, Br oder I steht, geschüttelt wird und organo-metallisches RMgX oder RLi und RMgX hinzugegeben werden.

2. Verfahren zur Herstellung einer etherischen Lösung oder Suspension eines Organo-Manganbromids nach Anspruch 1, dadurch gekennzeichnet, daß zunächst eine Lösung aus RMgX in Ether, wobei X für Cl, Br oder I steht, mit mindestens 1 Mol LiX gerührt wird, bis sich letzteres aufgelöst hat, worauf zu der Lösung MnBr₂ in einer molaren Menge hinzugegeben wird, die gleich ist wie die des hergestellten RMgX oder in deren Nähe liegt.

3. Verfahren nach Anspruch 1 oder 2, dadurch gekennzeichnet, daß das Lithiumhalogenid, insbesondere LiBr zuvor hergestellt wird in einem acyclischen aliphatischen flüssigen Ether, insbesondere unter Einwirken des Halogens auf Lithium in einem wasserfreien Ether unterhalb von Umgebungstemperatur, oder durch Einwirken eines organischen Lithiumderivats auf ein Alkyl- oder Allylhalogenid.

4. Verfahren nach einem der Ansprüche 1 bis 3, dadurch gekennzeichnet, daß das Manganbromid hergestellt wird durch Einwirken von Brom auf metallisches Mn-Pulver in einer organischen Flüssigkeit, vorzugsweise in einem aliphatischen Ester, insbesondere Ethylacetat, wobei das so hergestellte MnBr₂-Pulver eine spezifische Oberfläche von über 3,3 m²/g aufweist.

5. Anwendung des Verfahrens nach einem der Ansprüche 1 bis 4 zur Herstellung einer organischen Verbindung, dadurch gekennzeichnet, daß die Lösung oder Suspension, erhalten mit einer Vorstufe der genannten Verbindung, die in der Lage ist, mit RMnBr zu reagieren, gerührt wird.

6. Anwendung nach Anspruch 5, dadurch gekennzeichnet, daß die genannte Herstellung eine Carbonatisierung, Hydroxyalkylierung oder Acylierung der Vorstufe ist.

7. Anwendung nach Anspruch 6, dadurch gekennzeichnet, daß die Vorstufe ein organisches Säurechlorid R'-COCl oder ein gemischtes Anhydrid R'-COO-COR'' ist, wobei R' und R'' Kohlenwasserstoff-Gruppen darstellen und die hergestellte organische Verbindung ein Keton ist.

8. Anwendung nach Anspruch 7, dadurch gekennzeichnet, daß das Organo-Mangan folgendes ist:
(C₂H₅O)₂-CH-C≡C-Mn-Br oder CH₃-O-CH₂C≡C-Mn-Br und daß das Säurechlorid ClCO(CH₂)₅-O-CHO oder ClCO(CH₂)₅Cl ist.
